# EUROPEAN PATENT SPECIFICATION

(11) **EP 2 670 756 B1**
(45) Date of publication and mention of the grant of the patent: **21.06.2017**
(21) Application number: 11707171.2
(22) Date of filing: 08.03.2011
(51) Int. Cl.: C07D 498/18, C07F 7/18, A61K 31/435

(54) **PROCESS FOR MAKING EVEROLIMUS**
VERFAHREN ZUR HERSTELLUNG VON EVEROLIMUS
PROCÉDÉ POUR LA FABRICATION D'ÉVÉROLIMUS

(30) Priority: 04.02.2011 US 201161439830 P
(43) Date of publication of application: 11.12.2013
(73) Proprietor: Synthon BV, 6545 CM Nijmegen (NL)
(72) Inventor: LUTEN, Jordy, NL-6545 CM Nijmegen (NL); KELTJENS, Rolf, NL-6545 CM Nijmegen (NL); BENES , Michal, 19011 Prague 9 Prague (CZ); JANOUSEK, Vladimir, 19011 Prague 9 Prague (CZ); PÍS, Jaroslav, 19011 Prague 9 Prague (CZ)
(74) Representative: Mendivil Gil, Maria Dolores
(86) International application number: PCT/EP2011/053434
(87) International publication number: WO 2012/103959

(56) References cited:
- WO-A1-94/09010
- US-A1- 2003 125 800
- WHEELER T N ET AL: "Substrate specificity in short-chain phospholipid analogs at the active site of human synovial phospholipase A2.", JOURNAL OF MEDICINAL CHEMISTRY 25 NOV 1994 LNKD- PUBMED:7990112, vol. 37, no. 24, 25 November 1994 (1994-11-25), pages 4118-4129, XP002632693, ISSN: 0022-2623
- MOENIUS T ET AL: "Tritium labelling of RAD001- a new rapamycin derivative", JOURNAL OF LABELLED COMPOUNDS AND RADIOPHARMACEUTICALS, JOHN WILEY, CHICHESTER, GB, vol. 43, no. 2, 1 January 2000 (2000-01-01), pages 113-120, XP002335458, ISSN: 0362-4803, DOI: DOI:10.1002/(SICI)1099-1344(200002)43:2&LT ,113::AID-JLCR295&GT,3.0.CO,2- cited in the application
- Patrick G. Mcdougal ET AL: "A Convenient Procedure for the Monosilylation of Symmetric 1,n-Diols", J. Org. Chem, vol. 51, 1 January 1986 (1986-01-01), pages 3388-3390, XP055220088,

## Description

### BACKGROUND OF THE INVENTION

Everolimus, 40-O-(2-hydroxyethyl)-rapamycin of formula (1) is a pharmaceutically effective compound. It is a synthetic derivative of rapamycin (sirolimus) of formula (2), which is produced by a certain bacteria strain and is also pharmaceutically active.

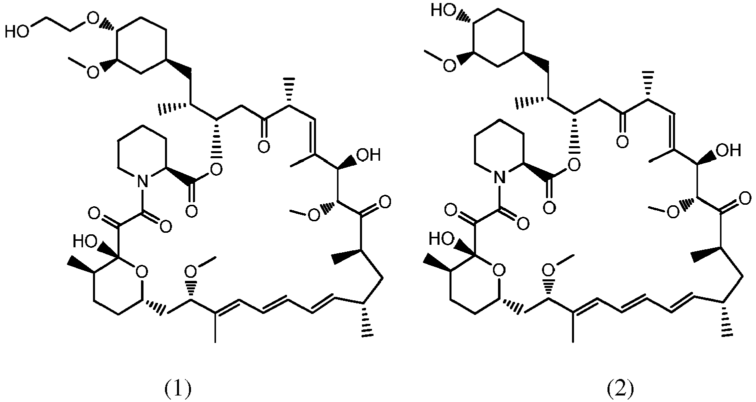

Everolimus differs from the parent rapamycin in that the carbon C₄₀ of rapamycin is substituted by a 2-hydroxyethoxy chain. Thereby the molecule becomes more polar, and purportedly has better oral bioavailability. Furthermore, the mechanism of action of everolimus in the human body is apparently slightly different than for rapamycin, which leads to broader therapeutic potential.

Everolimus is marketed under the brand name Certican for the prevention of rejection episodes following heart and kidney transplantation, and under the brand name Afinitor for treatment of advanced kidney cancer. Both products are tablets for oral administration; Certican is available in tablet strengths of between 0.1 to 1 mg, while Afinitor has been marketed in 2.5, 5 and 10 mg tablet strengths.

Everolimus is a white or almost white amorphous powder, which is almost insoluble in water. The molecule contains 15 chiral carbons and 4 substituted double bonds. The configuration on the chiral carbons and the double bonds correspond to rapamycin.

Due to its complicated macrolide chemical structure, everolimus is, similarly as the parent rapamycin, an extremely unstable compound. It is sensitive, in particular, towards oxidation, including aerial oxidation. In pharmaceutical compositions, everolimus is therefore stabilized by butylated hydroxytoluene (BHT). It is also unstable at temperatures higher than 25°C and at alkaline pH.

Everolimus and a process of making it have been disclosed in WO 94/09010 (US 5,665,772, EP 663916). The process principle is shown in the scheme below, wherein the abbreviation RAP-OH has been used as an abbreviation for the rapamycin structure of formula (2) above, L is a leaving group and P is an hydroxy-protective group.

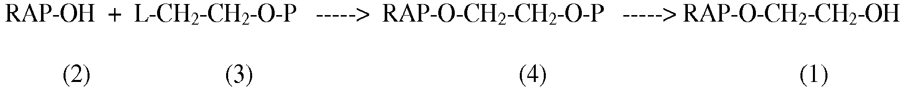

Specifically, the L- group is a trifluoromethanesulfonate (triflate) group and the protective group P- is typically a tert-butyldimethylsilyloxy- group. Accordingly, the known useful reagent of the above general formula (3) for making everolimus from rapamycin is 2-(tert-butyldimethylsilyloxy)ethyl triflate of formula (3A):

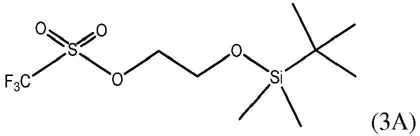

The synthesis of the compound (3A) was disclosed in Example 1 of US application 2003/0125800. It should be noted that the reaction solvent in the key step B of this synthesis has been omitted; however, further disclosure allows for estimation that such solvent is dichloromethane. Similarly also an article of Moenius et al. [J. Labelled Cpd. Radiopharm. 42, 29-41 (1999)] teaches that dichloromethane is the solvent in the reaction.

According to a known synthetic procedure disclosed in Example 8 of WO 94/09010 and in Example 1 of US application 2003/0125800, rapamycin (2) reacts in hot toluene and in the presence of 2,6-lutidine with a molar excess of the compound (3A), which is charged in several portions, to form the t-butyldimethylsilyl-protected everolimus (4A). This compound is isolated and deprotected by means of 1N aqueous HCl in methanol. Crude everolimus is then purified by column chromatography.

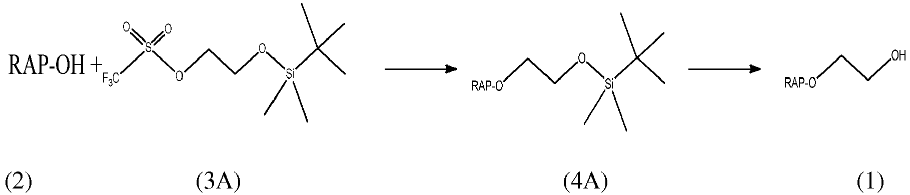

Yields both of the protected everolimus and of the final product have not been provided in the above documents. It appears that high molar excess of (3A) is necessary (4 equivalents in WO 94/09010, up to 40 equivalents in US Appl. 2003/0125800), which indicate either low reactivity of the (3A) or instability of (3A) during reaction conditions. Repetition of the process by the present inventors confirmed low yields of the process (about 17% yield of the compound (4A) according to WO 94/09010 and about 19% yield according to US Appl. 2003/0125800).

In a second article of Moenius et al. (J. Labelled Cpd. Radiopharm. 43, 113-120 (2000)), which used the above process for making C14-labelled and tritiated everolimus, a diphenyl-tert.butylsilyloxy-protective group was used as the alkylation agent shown as (3B) below. 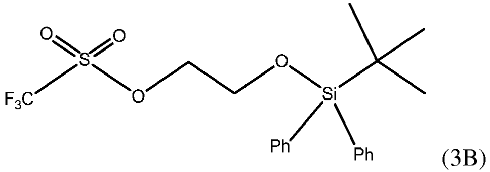 Only 8% yield of the corresponding compound (4B) 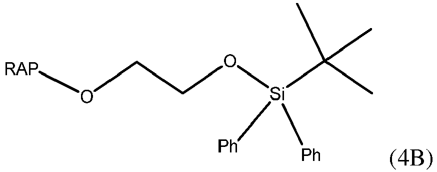 and 21% yield of the compound (1) have been reported. Thus, it appears that improvements in the process of making everolimus from rapamycin are necessary. In particular, a development of a process exhibiting higher yields and fewer impurities would be certainly appreciated.

### BRIEF DESCRIPTION OF THE INVENTION

The present invention relates to an improved process of making everolimus.

A first aspect of the invention relates to a process, which comprises a step of reacting rapamycin of formula (2) 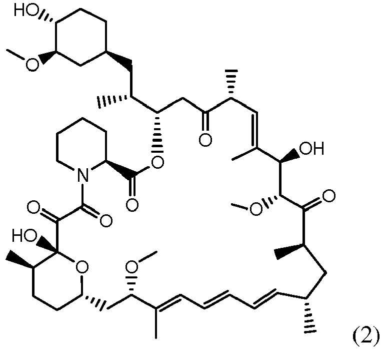 with 2-(t-hexyldimethylsilyloxy)ethyl triflate of formula (3C) 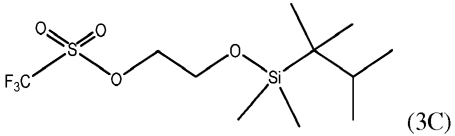 in an inert solvent, wherein the solvent is an aliphatic, cyclic or aromatic hydrocarbon of 5 to 10 carbon atoms, a chlorinated hydrocarbon of 1 to 6 carbon atoms, an aliphatic or cyclic ether of 4 to 10 carbon atoms and 1 to 3 oxygen atoms, and mixtures thereof, in the presence of aliphatic tertiary amine, to form 40-O-2-(t-hexyldimethylsilyloxy)ethylrapamycin of formula (4C), 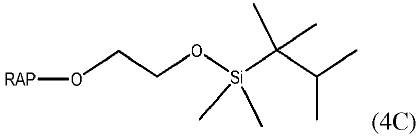 wherein "RAP" represents the residue of a 40-position O-substituted rapamycin.

The aliphatic tertiary amine can be tri-isobutylamine. The 40-O-2-(t-hexyldimethylsilyloxy)ethyl-rapamycin of formula (4C) is optionally isolated and/or extracted from the reaction mixture and can be additionally purified.

A second aspect of the invention relates to the deprotection of compound (4C) by an acid to form everolimus. The process comprises reacting a 40-O-2-(t-hexyldimethylsilyloxy)ethylrapamycin of formula (4C) 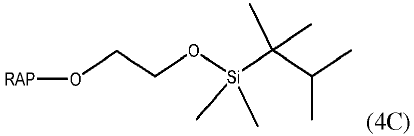 wherein RAP represents the residue of a 40-position substituted rapamycin, with an acid to form everolimus of formula (1):

RAP-O-CH₂-CH₂-OH (1)

wherein RAP represents the residue of a 40-position substituted rapamycin. The acid is typically a strong mineral acid or a strong organic acid, preferably hydrofluoric acid, hydrochloric acid, or trifluoroacetic acid. The deprotection reaction is often performed in an alcohol solvent, typically an alcohol having 1-5 carbon atoms.

A third aspect of the invention relates to a process, which comprises reacting 2-(t-hexyldimethylsilyloxy)ethanol of formula (5C): 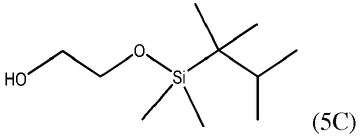 with trifluoromethane sulfonic acid or an activated derivative thereof, preferably of anhydride of the acid, in the presence of a base, to form 2-(t-hexyldimethylsilyloxy)ethyl triflate of formula (3C) 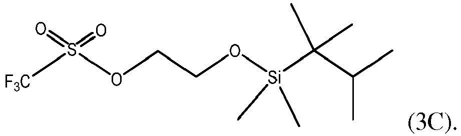 The reaction is preferably carried out in an alkane solvent, preferably having 5 to 12 carbons, such as pentane or hexane. The compound (3C) can be used in the above two aspects to ultimately form everolimus.

A fourth aspect of the invention relates to a compound 2-(t-hexyldimethylsilyloxy)ethyl triflate of formula (3C). The compound is useful as a reagent in making everolimus. In an advantageous mode, the compound is substantially free of salts, e.g., trifluoromethane sulfonic acid salts formed as a side product with the base.

A fifth aspect of the invention relates to a compound 40-O-2-(t-hexyldimethylsilyloxy)ethylrapamycin of formula (4C) and its use as a reagent in making everolimus.

### DETAILED DESCRIPTION OF THE INVENTION

The complex macrolide structure of rapamycin and everolimus allows several possible degradation pathways to occur at various positions of the molecule (oxidation, hydrolysis, racemization etc.). This degradation may occur not only during handling and storage of the product but, in an important aspect, during the process of making everolimus. While some reaction conditions may allow for the desired chemical reaction, they may also cause, on another site of the molecule, an undesired chemical change of the targeted macrolide.

Degradation and side products must be removed by quite complicated purification methods, incl. chromatography, which decrease the overall yield and are time and material consuming. Therefore, an industrially useful synthetic process should prevent the side reactions in maximum possible way.

The present invention relates to the reaction pathway leading to everolimus of formula (1), in which rapamycin of formula (2) is O-alkylated selectively on the position 40 by the compound of formula (3) below, in which L- is a suitable leaving group and P- is a suitable OH-protective group ,which typically is a silyl-protective group of formula

-Si (R1)(R2)(R3),

wherein R1, R2, R3 are independently a straight or branched alkyl group of 1 to 10 carbon atoms and/or a phenyl group.

The formed intermediate of formula (4) is then converted to everolimus by removal of the protective group P.

The process is illustrated in the scheme below, wherein RAP- represents the residue of a 40-position substituted rapamycin:

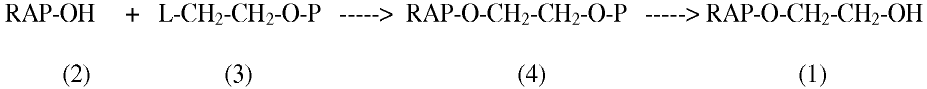

In accordance with the teaching above, proper selection of both the leaving group L- and the protective group P- is apparently of utmost importance. For instance, it could be expected that the O-alkylation step on the OH-group attached to carbon 40 of the rapamycin skeleton might be performed under essentially the same conditions as, e.g., on a cyclohexanol. However, common leaving groups for the O-alkylation reaction require relatively harsh reaction conditions so that the alkylation reaction of rapamycin would be accompanied with enormous amounts of side- and decomposition products. Thus, using extremely reactive trifluoromethane sulfonate (triflate) leaving group is a good option as it may alkylate the OH-group in position 40 under very mild conditions.

However, a reactive compound is also an unstable compound. It has been already discussed above that the yields of the alkylation of rapamycin with the compound of formula (3A) are relatively low - only about 20% - and the compound (3A), in a large molar excess, is charged into the reaction mixture in several portions. Both these features indicate that the compound (3A) has been decomposed in a large extent at the chosen reaction conditions. Stabilization of the compound (3A), preferably without a loss of reactivity, would be desirable, however no indication how to stabilize has been provided in the prior art.

It was now found that the compound (3A) may be successfully replaced in the step of alkylation of rapamycin by 2-(t-hexyldimethylsilyloxy)ethyl triflate of formula (3C) of the present invention. This compound may react with rapamycin under essentially similar reaction conditions, however it produces the protected everolimus of general formula (4) in better yields and with fewer impurities. For evidence, see Example 7 below.

For clarity, the "t-hexyl" as used in the present invention is an abbreviation for 2,3-dimethylbut-2-yl moiety.

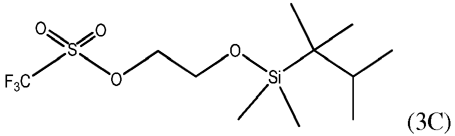

In an important aspect, while the use of t-hexyldimethylsilyl- protective group generates a more stable triflate, such group is still labile enough to be removed in the next step of everolimus synthesis under mild acidic conditions without destroying the product. For comparison, when a tri-isopropylsilyl protective group was tested in triflate compounds of the general formula (3), such group was proven as useful in making compound of general formula (4), but caused big problems (low yields, high amount of impurities) in the deprotection step as the deprotection was successful only under harsher conditions.

In summary, the use of 2-(t-hexyldimethylsilyloxy)ethyl triflate (3C) for making everolimus represents a better balance between stability and reactivity and can provide everolimus in higher yields than when using the 2-(tert-butyldimethylsilyloxy)ethyl triflate (3A) or the 2-(tert-butyldiphenylsilyloxy)ethyl triflate (3B) of the prior art.

The 2-(t-hexyldimethylsilyloxy)ethyl triflate of formula (3C) may be produced by a two step process.

In a first step, ethyleneglycol reacts with t-hexyldimethylsilylchloride in an inert solvent or, advantageously, without using a solvent, in the presence of a base, which may be a hydride base or, advantageously, is an amine base, yielding 2-(t-hexyldimethylsilyloxy)-ethanol of formula (5C).

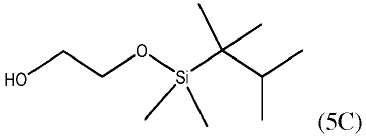

In the second step, the compound (5C) reacts with trifluoromethane sulfonic acid or an activated derivative thereof, e.g. with trifluoromethane sulfonic anhydride (triflic anhydride), in the presence of a base. The process may be carried out under conditions analogous to the process of making the known compound (3A), i.e. in dichloromethane. However, the present inventors found out that such reaction may advantageously proceed in an alkane solvent. The key advantage of this alkane solvent is that the second product of the reaction, a salt of triflic acid with the base, is insoluble in this solvent and may be removed by a simple filtration. To the contrary, the prior art dichloromethane solvent dissolves this salt, which must then be removed by a relatively complicated extraction.

Thus, the compound (5C) advantageously reacts with trifluoromethane sulfonic acid or an activated derivative thereof, e.g. with trifluoromethane sulfonic anhydride (triflic anhydride), in a C5-C12 alkane solvent, in a presence of a base, which preferably is an amine base, advantageously a tertiary amine base. The "alkane" in this particular consequence means a straight or branched or cyclic alkane as well as mixtures thereof, including mixtures thereof with an arene, e.g. benzene or toluene. Advantageously, the alkane is n-pentane, n-hexane or n-heptane.

The reaction temperature is typically ambient or lower than ambient, advantageously from -10 to -40°C. The formed insoluble triflic salt of the base is removed by filtration and the product is isolated from the filtrate, e.g. by evaporation of the solvent. After isolation, the compound may be obtained as an oily product. It should be also noted that it was found that removing the triflate salts by simple filtration, without using aqueous extraction, highly increases the stability of the silylated triflate, apparently due to avoiding formation of side products by hydrolysis during the extraction. The above process thus provides the compound of formula (3C) substantially free of amine salts of triflic acid (i.e., less than 10% of the original amount of salts is present after filtration), which is advantageous from stability point of view.

The quality, i.e., content and purity of the produced triflate (3C) in the isolated product should be advantageously determined before its use in the process of making everolimus. As the triflate is not itself detectable by suitable analytical methods based on UV detection, a derivatization method has been developed for monitoring this compound. This derivatization method is based on the reaction of the triflate (3C) with a derivatization agent, which preferably is an aromatic secondary amine, for instance N-methylaniline. The formed tertiary aromatic amine, which now bears a chromophor group, may then be detected and/or its content may be determined by a suitable analytical technique, e.g. by HPLC.

The same derivatization method may be used for analysing purity and/or stability of the triflate compound of formula (3C) during storage.

In the first step of the process of making everolimus of the present invention, the compound (3C) reacts with rapamycin in an inert solvent and in the presence of an aliphatic tertiary amine. The solvent may be an aliphatic, cyclic, or aromatic hydrocarbon of 5 to 10 carbon atoms, typically toluene, a chlorinated hydrocarbon of 1 to 6 carbon atoms, typically dichloromethane, an aliphatic or cyclic ether of 4 to 10 carbon atoms and 1 to 3 oxygen atoms, typically dimethoxyethane, and mixtures thereof. The inert solvent is advantageously anhydrous, typically containing less than 0.1 % of water. The solvent may be advantageously deaerated by an inert gas, e.g., by nitrogen or argon, and the reaction may advantageously proceed in the atmosphere of such inert gas. The aliphatic tertiary amine is typically a highly sterically hindered tertiary amine-type base, as such base is weakly nucleophilic due to sterical hindrance. Advantageously, the base is a tertiary aliphatic amine with at least two branched aliphatic groups. The branched aliphatic groups can be the same or different and typically have 3-8 carbon atoms. Examples include tris(2-methylpropyl)amine and N,N-diisopropylethylamine. The molar ratio of the compound (3C) to rapamycin preferably does not exceed 5:1, and typically is about 4:1, in some embodiments about 3:1 or even about 2:1. The compound (3C) is advantageously charged in one portion. The base is typically charged in a molar equivalent in respect to the compound (3C). The reaction temperature is typically an ambient or higher than ambient temperature, preferably between 25°C and 75°C. The reaction and elaboration of the reaction mixture may, in an advantageous arrangement, proceed under the absence of light.

In an advantageous mode, the course of the reaction is monitored by a suitable analytical method, e.g. by TLC or HPLC. In particular, the reaction should be monitored for the presence of the not yet reacted triflate (3C) as it was found that the presence of the unreacted triflate (3C) in the reaction mixture should be avoided in the next stage of elaboration of the reaction mixture. For reasons discussed above, the triflate is advantageously monitored by the derivatization method based on the reaction of the triflate (3C) with a derivatization agent, which preferably is a secondary aromatic amine, typically N-methylaniline.

The product of the reaction of compound (3C) with rapamycin is the compound of formula (4C), 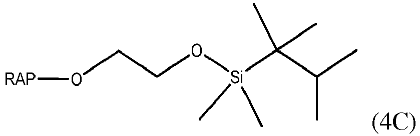 which may, optionally, be isolated from the reaction mixture. The reaction mixture is typically elaborated by an extraction with an aqueous base, drying, and removal of the solvent. The crude compound (4C) may be optionally purified, e.g., by chromatography, advantageously by column chromatography on a silica-packed column.

In the second step of the process of the present invention, the compound (4C) is subjected to a deprotection reaction, which removes the t-hexyldimethylsilyl- protective group. The deprotection is performed by reacting the compound (4C) with an acid in a suitable solvent. The solvent is typically an aliphatic alcohol of 1-5 carbon atoms, advantageously methanol. The acid is typically a strong mineral or organic acid, advantageously hydrofluoric acid, hydrochloric acid or trifluoroacetic acid. The acid may be charged as an aqueous solution. The temperature of the reaction is advantageously ambient. The reaction may be advantageously performed under inert atmosphere and in the absence of light and may be monitored by a suitable analytical method, e.g. by TLC or HPLC.

After termination of the reaction, the reaction mixture is neutralised by a base, advantageously by an aqueous base, more advantageously by a solution of sodium bicarbonate, and extracted by a water-immiscible organic solvent. The product is isolated from the organic phase, optionally after washing and drying, by removal of the extraction solvent. The crude product may be purified, e.g., by chromatography, advantageously by column chromatography on a silica-packed column.

Thus, in summary, the novel compounds of formulas (3C) and (4C) may be used as reagents in a process for making everolimus. By using these compounds, the process of making everolimus may be more effective than processes disclosed in the prior art.

The novel compounds may accordingly be used in making derivatives of everolimus, particularly those, which use everolimus as the starting material. In a particular aspect, the compound (4C) may be used for protection or masking the 40-O-(2-hydroxyethyl)- group on the rapamycin ring in a process of making derivatives of everolimus.

The invention is further illustrated by the following examples but they should not be construed as limiting the scope of this invention in any way.

### EXAMPLES

### Example 1: 2-((2,3-dimethylbut-2-yl)dimethylsilyloxy)ethyl trifluoromethanesulfonate

### Step 1 - 2-(2,3-dimethylbut-2-yl)dimethylsilyloxy)ethanol

In a 1 L round-bottomed flask ethane-1,2-diol (100.02 g, 1.611 mol) and chloro(2,3-dimethylbutan-2-yl)dimethylsilane (43.40 g, 243 mmol) were mixed to give two liquid colorless non-miscible layers. The flask was placed into a water bath set at 30°C. Pyridine (299 ml, 3.699 mol) was added within approx. 30 sec under stirring, whereby a solution was formed. The reaction mixture was stirred for 1 hr.

After that the mixture was neutralized with 2.00 g of sodium acetate and 12.75 g of sodium carbonate. The mixture was stirred for 20 min at 30°C and left overnight.

Pyridine was distilled off using vacuum (vacuum 50 mbar, bath temperature 50°C - 75°C). The residue (yellow suspension) was cooled down and partitioned between water (200 ml) and methyl t-butylether (200 ml). The layers were separated and the organic layer was washed with water (200 ml) and dried over Na₂SO₄ (20 g). The solvents were removed by vacuum distillation (330 to 160 mbar, bath temperature 60°C) to give the crude product as a yellow oil (57.5g). The crude product was further purified by vacuum distillation (vacuum 25 to 2 mbar, batch temperature 90°C to 100°C). Two fractions containing product were collected: fraction 2 (24.10 g, boiling point 50-55°C / 2-3 mbar) and fraction 3 (17.40 g, boiling point 55-60°C / 2-3 mbar). In total 41.2 g (82.9% of the theoretical yield) was obtained.

### Step 2

To a 100 mL flask charged with trifluoromethanesulfonic anhydride (0.889 ml, 5.28 mmol) in CH₂Cl₂ (20 ml) there was added dropwise at -8°C 2-((2,3-dimethylbut-2-yl)dimethylsilyloxy)ethanol (0.9 g, 4.40 mmol) and N,N-diisopropylethylamine (1.151 ml, 6.61 mmol). An orange solution formed and was stirred for 30 min at -8°C. The orange solution was concentrated to a brown/orange solid, which was purified using column chromatography on silica gel (CH2Cl2, Rf 0.8). After concentration of the right fraction, the product was obtained as an oil.

### Example 2: 2-((2,3-dimethylbut-2-yl)dimethylsilyloxy)ethyl trifluoromethane-sulfonate

The reaction was carried out under nitrogen. In a 1 L round-bottomed flask, 2-((2,3-dimethylbut-2-yl)dimethylsilyloxy)ethanol (18.29 g, 89 mmol) and N-ethyl-N-isopropyl-2-aminopropane (12.14 g, 94 mmol) were stirred in n-heptane (366 ml) to give a colorless solution. The solution was cooled down under stirring (bath temperature -30 °C). A 250 mL dropping funnel was charged with a solution of trifluoromethanesulfonic anhydride (25.20 ml, 89 mmol) in n-heptane (183 ml). This solution was added dropwise over 15 min to the reaction mixture (white precipitate started to form immediately) while maintaining the temperature of the reaction mixture between -33°C and -25°C. The reaction mixture was stirred at a temperature between - 30°C and -15°C for 120 minutes. The precipitate was filtered off. The filtrate was filtered through silica gel (65 g), the silica gel was washed with heptane (1,000 ml). The combined liquid was evaporated to give the desired product.

The total yield was 26.07 g (86.6% of the theoretical yield).

### Example 3: Derivatization method for the determination of 2-((2,3-dimethylbut-2-yl)dimethylsilyloxy)ethyl trifluoromethane sulfonate

### Model sample:

2-((2,3-dimethylbut-2-yl)dimethylsilyloxy)ethanol (232 mg, purity 97.9%, 1.112 mmol) was charged into a reaction flask and pentane (3 ml) was added followed by N-ethyl-N-isopropyl-2-aminopropane (0.203 ml, 1.192 mmol). The reaction mixture was cooled down to 0°C. Trifluoromethanesulfonic anhydride (320 mg, 1.135 mmol) was dissolved in 3 ml of pentane and this solution was added to the stirred reaction mixture over 5 minutes using a syringe while maintaining the temperature at 0 °C. The reaction mixture was stirred at 0°C for 30 min.

### Derivatization:

10 ml of solution of derivatization agent (1M solution of N-methylaniline in acetonitrile:dichloromethane 3:1 v/v) is added to the model sample at once using a pipette and the reaction flask with the mixture is immersed in a water bath (room temperature). The derivatization mixture is stirred at room temperature for 15 min. The content of the reaction flask is transferred to a 50 ml volumetric flask and the volume is made up with acetonitrile. 1.0 ml of this solution is transferred to a 25 ml volumetric flask and the volume is made up with acetonitrile. This sample (10 µl) is injected onto the HPLC and analyzed. The recovery as determined by HPLC is 355mg (95% of the theoretical yield).

### HPLC settings:

| | | |
|---|---|---|
| Analytical column: | Gemini C18 110 A, 150 x 4.6 mm, 5 µm | |
| Guard column: | n.a. | |
| Column temperature | ambient | |
| Flow rate | 1 ml / min | |
| Detection | UV 255 nm | |
| Injection volume | 10 µl | |
| | | |

| Gradient program: | Time [min] | conc. of B [%] |
|---|---|---|
| | 0 | 50 |
| | 5 | 55 |
| | 11 | 95 |
| | 18 | 95 |
| | 19 | 50 |
| | 24 | 50 |

Mobile phase A: 20 mM ammonium formiate buffer pH 4.0 (1.26 g of NH₄CO₂H in 1 L of water, pH adjusted to pH=4.0 by (diluted) formic acid).
Mobile phase B: Acetonitrile

### Example 4: Use of derivatization method for testing stability of the triflate (3C)

### a] Stability of the triflate in dichloromethane

To a 10 mL reaction flask 2-((2,3-dimethylbut-2-yl)dimethylsilyloxy)ethanol (200 mg, 0.979 mmol) and triisobutylamine (181 mg, 0.979 mmol) in CH₂Cl₂ (3 ml) were added. The reaction mixture was cooled down to -10°C under nitrogen. In a 5 mL flask, a solution of trifluoromethanesulfonic anhydride (276 mg, 0.979 mmol) in CH₂Cl₂ (3.00 ml) was prepared. This solution was added dropwise to the reaction mixture during 5 min. The reaction mixture (solution) was stirred for 20 min at -10°C. The temperature of the reaction mixture was adjusted to room temperature and the content of triflate was determined by the HPLC method at several time points. The results are presented in the following table (the content of triflate is expressed as % of the theoretical yield corrected for the purity of the starting material 2-((2,3-dimethylbut-2-yl)dimethylsilyloxy) -ethanol).

| Time (hours) | Yield of triflate (% of theor. yield) |
|---|---|
| 0 | 81.89 |
| 1 | 72.52 |
| 2 | 71.50 |
| 4 | 68.48 |
| 6 | 67.68 |

### b) Stability of triflate in pentane

To a reaction flask 2-((2,3-dimethylbut-2-yl)dimethylsilyloxy)ethanol (400 mg, 1.957 mmol) and triisobutylamine (0.474 ml, 1.957 mmol) in 6 ml of pentane were added. The reaction mixture was cooled down to -10°C under nitrogen. In a flask was prepared solution of trifluoromethanesulfonic anhydride ((552 mg, 1.957 mmol) in pentane (6 ml). This solution was added dropwise to the reaction mixture during 5 min. The reaction suspension was stirred at - 10°C for 20 min and the solids were filtered off and washed with 5 ml of pentane. The temperature of the filtrate was adjusted to room temperature and the content of triflate was determined by the HPLC method at several time points. The results are presented in the following table (the content of triflate is expressed as % of the theoretical yield corrected for the purity of the starting material 2-((2,3-dimethylbut-2-yl)dimethylsilyloxy)ethanol).

| Time (hours) | Yield of triflate (% of theor. yield) |
|---|---|
| 0 | 89.05 |
| 1 | 89.09 |
| 2 | 89.54 |
| 4 | 90.28 |
| 6 | 90.29 |

### Example 5: 40-O-[2-((2,3-dimethylbut-2-yl)dimethylsilyloxy)ethyl]rapamycin

In a 100 mL flask, Rapamycin (6 g, 6.56 mmol) was dissolved in dimethoxyethane (4.2 ml) and toluene (24 ml) to give a white suspension and the temperature was raised to 70°C. After 20 min, N,N-diisopropylethylamine (4.56 ml, 27.6 mmol) and 2-((2,3-dimethylbutan-2-yl)dimethylsilyloxy)ethyl trifluoromethanesulfonate (8.83 g, 26.3 mmol) were added in 2 portions with a 2 hr interval at 70°C. The mixture was stirred overnight at room temperature, then diluted with EtOAc (40 ml) and washed with sat. NaHCO₃ (30 ml) and brine (30 ml). The organic layer was dried with Na₂SO₄, filtered and concentrated. The crude product was chromatographed on a silica gel column (EtOAc/heptane 1/1; yield 4.47 g).

### Example 6: 40-O-(2-hydroxyethyl)-rapamycin [everolimus]

In a 100 mL flask, 40-O-[2-((2,3-dimethylbut-2-yl)dimethylsilyloxy)ethyl]rapamycin (4.47 g, 4.06 mmol) was dissolved in methanol (20 ml) to give a colorless solution. At 0°C, 1N aqueous hydrochloric acid (2.0 ml, 2.0 mmol) was added and the mixture was stirred for 90 min. The reaction was followed by TLC (ethyl acetate/n-heptane 3:2) and HPLC. Then 20 ml of saturated aqueous NaHCO₃ were added, followed by 20 ml of brine and 80 ml of ethyl acetate. The phases were separated and the organic layer was washed with saturated aqueous NaCl until pH 6/7. The organic layer was dried by Na₂SO₄, filtered and concentrated to yield 3.3 g of the product.

### Example 7: Preparation of silyloxyethylated rapamycin - comparative results A] 40-O-[2-(t-Butyldimethylsil)oxy]ethyl-rapamycin - Reproduction of WO94/09010,

### Example 8 (experiment was performed on 10% scale of said example 8)

Reagents with following water content were used: toluene (0.002% of H₂O) and 2,6-lutidine (0.015% of H₂O). A solution of rapamycin (0.914 g, 1.00 mmol) and 470 µl of 2,6-lutidine (4 mmol) in 3 ml of dry toluene was warmed to 60°C. Then the solution of 2-(tert-butyldimethylsilyloxy)ethyl triflate (0.795 g, assay 77.6%, corresponds to 0.617g of 100% material, 2.00 mmol) and 235 µl of 2,6-lutidine (2 mmol) in 2 ml of dry toluene were added. This mixture was stirred for 1.5 hr at 60°C.

Then the solution of 2-(tert-butyldimethylsilyloxy)ethyl triflate (0.397 g, assay 77.6%, corresponds to 0.308 g of 100% material, 1.00 mmol) and 120 µl of 2,6-lutidine (1 mmol) in 2 ml of dry toluene was added. The mixture was stirred for 1.5 hr at 60°C.

Then the solution of 2-(tert-butyldimethylsilyloxy)ethyl triflate (0.397 g, assay 77.6%, corresponds to 0.308 g of 100% material, 1.00 mmol) and 120 µl of 2,6-lutidine (1 mmol) in 2 ml of dry toluene was added. The mixture was stirred for 2 hr at 60 °C. After that, the suspension was cooled down to 20-25°C and the solids were filtered off and washed with 2 ml of toluene. The conversion was checked by HPLC: the reaction mixture contains 80.2% of rapamycin and 18.3% of product (area %).

The filtrate was diluted with EtOAc (25 ml) and washed with a saturated NaHCO₃ (2x25 ml) and a saturated NaCl solution (1x25ml). The organic layer was dried with Na₂SO₄, filtered and concentrated. The residue (m = 1.23g) was added to a silica gel column and was eluted with a mixture of hexane:EtOAc (40:60 v/v). Fractions were collected, analysed by HPLC and fractions of similar purity were combined. After evaporation of the solvents the product was obtained in two portions: 0.15601 g, purity 98.19%, corresponds to 153.186 mg of 100% material and 0.05008 g, purity 59.49%, corresponds to 29.793 mg of 100% material. The total yield calculated as 100% material was 182.979 mg, 17.1% of the theoretical yield.

### B] 40-O-[2-(t-Butyldimethylsil)oxy]ethyl-rapamycin - Reproduction of US 2003/0125800, Example 1, step C

Reagents with following water content were used: toluene (0.002% of H₂O) and 2,6-lutidine (0.015% of H₂O).

A solution of rapamycin (400 mg, 0.438 mmol) and 1.9 ml of 2,6-lutidine (16.31 mmol) in 10 ml of toluene was warmed to 55-57°C. Then the solution of 2-(tert-butyldimethylsilyloxy)ethyl triflate (3.183 g, assay 77.6%, corresponds to 4.47 g of 100% material, 8 mmol, 18.3 eq on rapamycin) and 940 µl of 2,6-lutidine (8.07 mmol) in 1 ml of toluene were added. This mixture was stirred for 1.5 hr at 55-57°C.

Then 480 µl of 2,6-lutidine (4.12 mmol) was added to the reaction mixture and immediately thereafter 2-(tert-butyldimethylsilyloxy)ethyl triflate (1.593 g, assay 77.6%, corresponds to 1.236 g of 100% material, 4 mmol, 9.16 eq. on rapamycin). This mixture was stirred for 1 hr at 55-57°C.

After that, the 480 µl of 2,6-lutidine (4.12 mmol) was added to the reaction mixture and immediately thereafter 2-(tert-butyldimethylsilyloxy)ethyl triflate (1.593 g, assay 77.6%, corresponds to 1.236 g of 100% material, 4 mmol, 9.16 eq. on rapamycin). This mixture was stirred for 1.5 hr at 55-57°C. The reaction suspension was cooled down to 20-25°C. The solids were filtered off and washed with 5 ml of toluene. The conversion was checked by HPLC: the reaction mixture contains 83.7% of rapamycin and 15.2% of product (area %).

The filtrate was washed with a saturated NaHCO₃ solution (2x60 ml) and a saturated NaCl (1x30ml) solution. The organic layer was dried with Na₂SO₄, filtered and concentrated (0.68 g).

The crude product was added to a silica gel (16 g) column and was eluted with a mixture of hexane:EtOAc (40:60 v/v). Fractions were collected, analysed by HPLC and fractions of similar purity were combined. After evaporation of the solvents the product was obtained in two portions: 0.09102 g, purity 93.9%, corresponds to 85.47 mg of 100% material and 0.0127 g, purity 43.65%, corresponds to 5.544 mg of 100% material. The total yield calculated as 100% material was 101.134 mg, 19.4% of the theoretical yield.

### C] 40-O-[2-((2,3-dimethylbut-2-yl)dimethylsilyloxy)ethyl]rapamycin

Rapamycin (300 mg, 0.328 mmol), toluene (1.2 ml), 1,2-dimethoxyethane (0.20 ml) and N-ethyl-N-isopropylpropan-2-amine (148 µl, 0.863 mmol) were added to 3 ml pear flask and the mixture was heated to 65°C finally giving a clear solution. After 5 min 2-((2,3-dimethylbutan-2-yl)dimethylsilyloxy)ethyl triflate (276 mg, assay 79.8% 0.655 mmol) was added at once to the solution. The reaction mixture was stirred at 65°C for 4 hours and samples were taken at the following time point intervals to monitor reaction progress.

| Reaction time: | Rapamycin (area %) | Product (area %) |
|---|---|---|
| 2 hours | 34.2% | 55.5% |
| 3 hours | 28.2% | 60.6% |
| 4 hours | 21.8% | 63.1% |

The reaction mixture was cooled down. The weight of the reaction mixture was 1.778 g. The reaction mixture was diluted with EtOAc (10 ml), transferred to a separatory funnel and washed with 10 ml saturated NaHCO₃ and NaCl (10 ml; 10% solution). The organic layer was dried with MgSO₄, filtered and evaporated under vacuum (bath temperature 35°C) to give crude product 0.520 g.

The crude product was purified by column chromatography (10 g silica gel, eluent, heptane-EtOAc 70:30). Fractions (10 ml) were collected, analysed and fractions of similar purity were combined. After evaporation of the solvents the product was obtained 166 mg, purity 97.3 %, corresponds to 161.52 mg of 100% material, 44.7% of the theoretical yield.

The invention having been described it will be obvious that the same may be varied in many ways and all such modifications are contemplated as being within the scope of the invention as defined by the following claims.

## Claims

1. A process, which comprises:
a step of reacting rapamycin of formula (2) 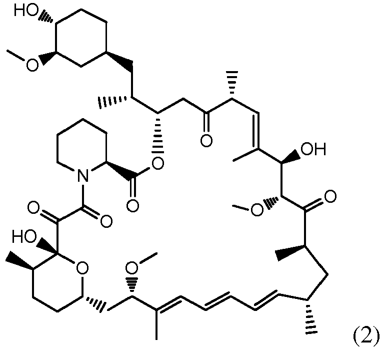 with 2-(t-hexyldimethylsilyloxy)ethyl triflate of formula (3C) 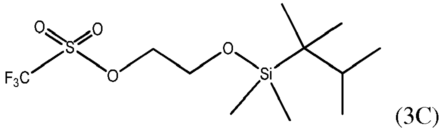 in an inert solvent, wherein the solvent is an aliphatic, cyclic or aromatic hydrocarbon of 5 to 10 carbon atoms, a chlorinated hydrocarbon of 1 to 6 carbon atoms, an aliphatic or cyclic ether of 4 to 10 carbon atoms and 1 to 3 oxygen atoms, and mixtures thereof, in the presence of an aliphatic tertiary amine to form 40-O-2-(t-hexyldimethylsilyloxy)-ethylrapamycin of formula (4C) 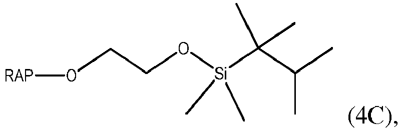 wherein RAP represents the residue of a 40-position O-substituted rapamycin

2. The process according to claim 1 wherein the solvent is selected from toluene, dichloromethane, dimethoxyethane and mixtures thereof.

3. The process according to claims 1 or 2, wherein the molar ratio of the compound of formula (3C) in respect to the compound of formula (2) is less than 5:1.

4. The process according to claims 1-3, wherein the reaction is monitored for the presence of the compound of formula (3C), by a derivatization method based on the reaction of the compound (3C) with a secondary aromatic amine.

5. The process according to claim 1-4, which further comprises isolating or extracting said compound of formula (4C) from the reaction mixture.

6. The process according to any of claims 1-5, which further comprises reacting the compound (4C) with an acid to form everolimus of formula (1):

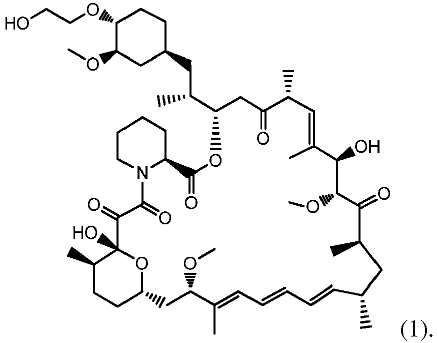

7. The process according to claim 6, wherein the acid is hydrofluoric acid, hydrochloric acid, or trifluoroacetic acid.

8. A compound 2-(t-hexyldimethylsilyloxy)ethyl triflate of formula (3C)

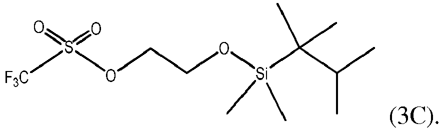

9. A process of monitoring the quality of the compound of general formula (3C), comprising a derivatization reaction of the compound of formula (3C) with an aromatic secondary amine and a detection and/or content determination of the product of the derivatization reaction by a suitable analytical technique.

10. A compound 40-O-2-(t-hexyldimethylsilyloxy)ethylrapamycin of formula (4C) 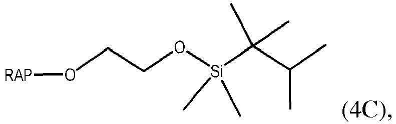 wherein RAP represents the residue of a 40-position O-substituted rapamycin.

11. Use of the compound of formula (3C), (4C) and/or (5C) in a process for making everolimus.

## Patentansprüche

1. Verfahren, dass umfasst:
einen Schritt des Umsetzens von Rapamycin der Formel (2) 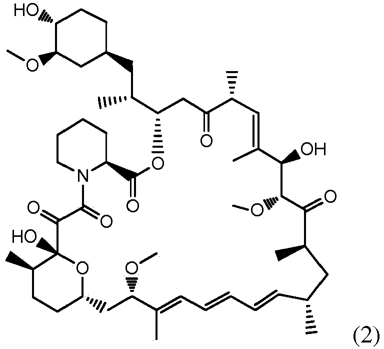 mit 2-(t-Hexyldimethylsilyloxy)ethyltriflat der Formel (3C) 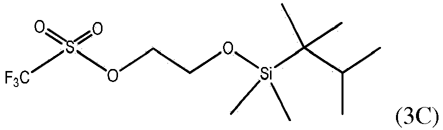 in einem inerten Lösungsmittel, wobei das Lösungsmittel ein aliphatischer, cyclischer oder aromatischer Kohlenwasserstoff von 5 bis 10 Kohlenstoffatomen, ein chlorierter Kohlenwasserstoff von 1 bis 6 Kohlenstoffatomen, ein aliphatischer oder cyclischer Ether von 4 bis 10 Kohlenstoffatomen und 1 bis 3 Sauerstoffatomen, und Gemische davon ist, in der Gegenwart eines aliphatischen tertiären Amins, um 40-O-2-(t-Hexyldimethylsilyloxy)ethylrapamycin der Formel (4C) zu bilden 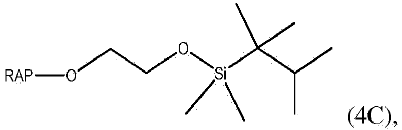 wobei RAP den Rest eines in 40-Position O-substituierten Rapamycins darstellt.

2. Verfahren nach Anspruch 1, wobei das Lösungsmittel aus Toluol, Dichlormethan, Dimethyoxyethan und Gemischen davon ausgewählt ist.

3. Verfahren nach Ansprüchen 1 oder 2, wobei das molare Verhältnis der Verbindung der Formel (3C) bezüglich der Verbindung der Formel (2) weniger als 5:1 beträgt.

4. Verfahren nach Ansprüchen 1-3, wobei die Reaktion auf die Gegenwart der Verbindung der Formel (3C) durch ein Derivatisierungsverfahren verfolgt wird, das auf der Reaktion der Verbindung (3C) mit einem sekundären aromatischen Amin basiert.

5. Verfahren nach Anspruch 1-4, das weiterhin Isolieren oder Extrahieren der Verbindung der Formel (4C) aus dem Reaktionsgemisch umfasst.

6. Verfahren nach einem beliebigen der Ansprüche 1-5, das weiterhin Umsetzen der Verbindung (4C) mit einem Säure umfasst, um Everolimus der Formel (1) zu bilden:

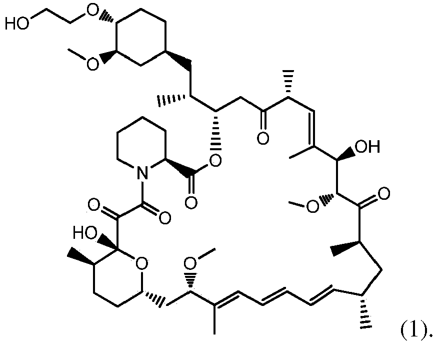

7. Verfahren nach Anspruch 6, wobei die Säure Flusssäure, Salzsäure oder Trifluoressigsäure ist.

8. Verbindung 2-(t-Hexyldimethylsilyloxy)ethyltriflat der Formel (3C)

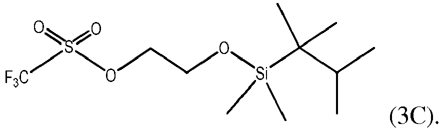

9. Verfahren zum Verfolgen der Qualität der Verbindung der allgemeinen Formel (3C), umfassend eine Derivatisierungsreaktion der Verbindung der Formel (3C) mit einem aromatischen sekundären Amin und einen Nachweis und/oder eine Gehaltsbestimmung des Produkts der Derivatisierungsreaktion durch eine geeignete Analysetechnik.

10. Verbindung 40-O-2-(t-Hexyldimethylsilyloxy)ethylrapamycin der Formel (4C) 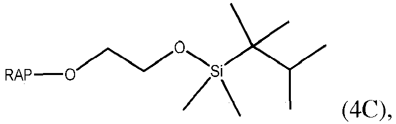 wobei RAP den Rest eines in 40-Position O-substituierten Rapamycins darstellt.

11. Verwendung der Verbindung der Formel (3C), (4C) und/oder (5C) in einem Verfahren zum Herstellen von Everolimus.

## Revendications

1. Un procédé qui comprend :
une étape de réaction de la rapamycine de formule (2) 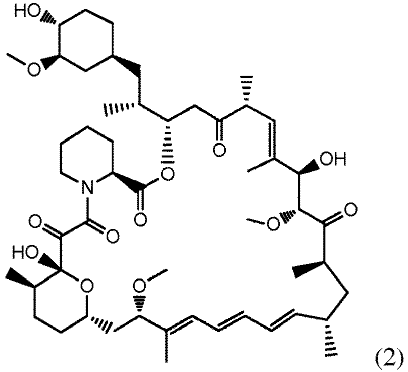 avec le triflate de 2-(t-hexyldiméthylsilyloxy)éthyle de formule (3C) 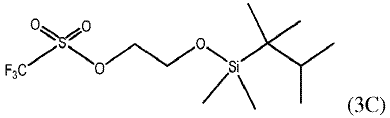 dans un solvant inerte, dans lequel le solvant est un hydrocarbure aliphatique, cyclique ou aromatique de 5 à 10 atomes de carbone, un hydrocarbure chloré de 1 à 6 atomes de carbone, un éther cyclique ou aliphatique de 4 à 10 atomes de carbone et de 1 à 3 atomes d'oxygène, et des mélanges de ceux-ci,
en présence d'une amine tertiaire aliphatique pour former le 40-O-2-(t-hexyl-diméthylsilyloxy)éthylrapamycine de formule (4C) 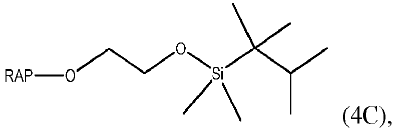 dans laquelle RAP représente le résidu d'une rapamycine O-substituée en position 40.

2. Le procédé selon la revendication 1, dans lequel le solvant est choisi parmi le toluène, le dichlorométhane, le diméthoxyéthane et des mélanges de ceux-ci.

3. Le procédé selon la revendication 1 ou 2, dans lequel le rapport molaire composé de formule (3C)/composé de formule (2) est inférieur à 5/1.

4. Le procédé selon les revendications 1-3, dans lequel la réaction est contrôlée, quant à la présence du composé de formule (3C), par un procédé de dérivatisation basé sur la réaction du composé (3C) avec une amine aromatique secondaire.

5. Le procédé selon l'une des revendications 1-4, qui comprend en outre l'isolation ou l'extraction dudit composé de formule (4C) du mélange réactionnel.

6. Le procédé selon l'une quelconque des revendications 1-5, qui comprend en outre la réaction du composé (4C) avec un acide pour former l'évérolimus de formule (1) :

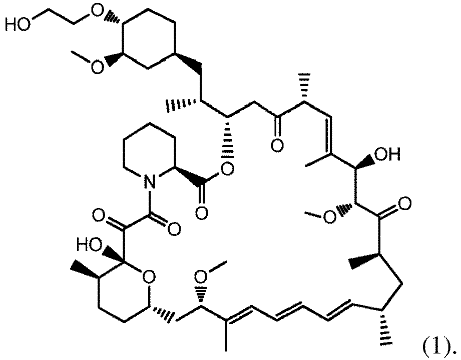

7. Le procédé selon la revendication 6, dans lequel l'acide est l'acide fluorhydrique, l'acide chlorhydrique ou l'acide trifluoroacétique.

8. Un composé consistant en triflate de 2-(t-hexyldiméthylsilyloxy)éthyle de formule (3C)

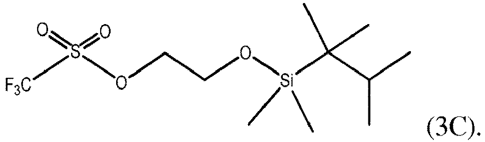

9. Un procédé de contrôle de la qualité du composé de formule générale (3C), comprenant une réaction de dérivation du composé de formule (3C) avec une amine aromatique secondaire et la détection et/ou détermination de la teneur du produit de la réaction de dérivation par une technique analytique appropriée.

10. Un composé consistant en 40-O-2-(t-hexyldiméthylsilyloxy)éthylrapamycine de formule (4C) 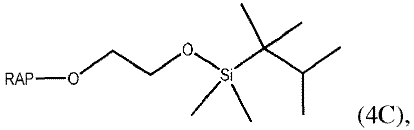 dans laquelle RAP représente le résidu d'une rapamycine O-substituée en position 40.

11. Utilisation du composé de formule (3C), (4C) et/ou (5C) dans un procédé de fabrication d'évérolimus.
